# EUROPÄISCHE PATENTSCHRIFT

(11) **EP 0 804 176 B1**
(45) Veröffentlichungstag und Bekanntmachung des Hinweises auf die Patenterteilung: **25.04.2001**
(21) Anmeldenummer: 95911253.3
(22) Anmeldetag: 23.02.1995
(51) Int. Cl.: A61K 31/04

(54) **TRANSDERMALE DARREICHUNGSFORM VON NITROGLYCERIN ZUR VERHINDERUNG UNERWÜNSCHTER WEHENTÄTIGKEIT**
TRANSDERMAL PRESENTATION OF NITROGLYCERIN FOR PREVENTING UNDESIRED LABOUR
PRESENTATION TRANSDERMIQUE DE NITROGLYCERINE POUR LA PREVENTION DES CONTRACTIONS INDESIRABLES

(30) Priorität: 28.02.1994 DE 4406332
(43) Veröffentlichungstag der Anmeldung: 05.11.1997
(73) Patentinhaber: SCHWARZ PHARMA AG, 40789 Monheim (DE)
(72) Erfinder: WOLFF, Hans-Michael, D-40789 Monheim (DE); SCHACHT, Dietrich, D-50935 Köln (DE); FEELISCH, Martin, D-40699 Erkrath (DE); RAMSAY, Bruce, Academic Dept. of Obstetrics, 369 Fulham Road, London SW10 9MH (GB); MARTIN, John, Francis, The Wellcome Foundation Ltd, Beckenham, Kent BR3 3BS (GB); LEES, Christoph, Christopher, Dept. of Obstetrics, Denmark Hill, London SE5 9RS (GB); DE BELDER, Adam, Julian, Dept. of Cardiology, London SE5 9RS (GB)
(86) Internationale Anmeldenummer: EP9500654
(87) Internationale Veröffentlichungsnummer: WO9522964

(56) Entgegenhaltungen:
- EP-A- 0 464 573
- LANCET, Bd. 343, Nr. 8909, 1994 Seiten 1325-1326, C. LEES ET AL. 'Arrest of preterm labour and prolongation of gestation with glyceryl trinitate, a nitric oxide donor.'

## Beschreibung

Die Erfindung betrifft die Verwendung von Nitroglycerin zur Herstellung eines Arzneimittels in einer transdermalen Applikationsform zur Verhinderung und Regulierung unerwünschter Wehentätigkeit bei Säugern.
Nitroglycerin, auch als Glyceroltrinitrat (GTN) bezeichnet, ist eine seit langem bekannte chemische Substanz mit vielfältigen Anwendungen. Chemisch wird GTN mit 1,2,3-Propantrioltrinitrat beschrieben. Insbesondere ist Nitroglycerin ein wertvoller Wirkstoff zur Behandlung von koronaren Herzkrankheiten. Dieser Bedeutung entsprechend stehen inzwischen viele pharmazeutisch technische Formulierungen zur Verfügung. Die galenischen Zubereitungen dieser Substanz umfassen inzwischen Tabletten, Kapseln, Salben, Lösungen Infusionslösungen, Sprays und Wirkstoffpflaster zur transdermalen Applikation.

Überraschenderweise wurde nun gefunden, daß die Verwendung von Nitroglycerin in einer transdermalen Applikationsform die unerwünschte Wehentätigkeit bei Säugern verhindert.

Studien bei Tieren und Menschen zeigten, daß die Wehentätigkeit durch diese galenische Zubereitungsform von Nitroglycerin unter gezielter Verwendung von Nitroglycerin beim jeweiligen Probanden gestoppt werden konnte.
Nachfolgend soll die an Menschen durchgeführte Studie die Erfindung näher erläutern.

Die Studie erfaßte 18 schwangere Frauen, die jeweils vorzeitige Wehentätigkeit aufwiesen. Ihre Schwangerschaften waren bereits fortgeschritten, denn sie waren bereits zwischen 25 und 34 Wochen schwanger. Die jeweils verwendeten GTN-Pflaster wurden an Stelle von Ritodrin, einem wehenhemmenden Wirkstoff, appliziert. Sämtliche schwangeren Probanden wiesen die folgenden Merkmale auf:
- die Fruchtblasen waren noch nicht geplatzt;
- vorzeitige Blutungen hatten nicht eingesetzt;
- die Föten wiesen keine Abnormitäten auf.

Die Patientinnen erhielten pro Tag bis zu 2 GTN-Pflaster gleichzeitig ä 10 bzw. 10,8 mg 1,2,3-Propantrioltrinitrat. Die Pflasterfläche betrug 32 cm² bei Verwendung von DEPONIT® 10 bzw. 16 cm² bei Verwendung eines neuartigen GTN-Pflasters der Fa. SCHWARZ PHARMA AG. Dieses letztgenannte Pflaster enthält das Nitroglycerin in einer Klebemasse auf Basis eines vernetzten Acrylat-Vinylacetat-Copolymerisates.

Es waren keine Unterschiede im verwendungsgemäßen Erfolg festzustellen. Beide Pflaster verhinderten vorzeitige Wehentätigkeit, stoppten vorzeitige Wehentätigkeit bzw. beendeten unregelmäßige Wehentätigkeit.

Tabellen 1 und 2 zeigen, daß Nitroglycerin in einer transdermalen galenischen Zubereitung unerwünschte vorzeitige Wehentätigkeit bei schwangeren Säugern verhindert.

Den Tabellen ist zu entnehmen, daß die Wehentätigkeit nach Applikation der Pflaster gestoppt wurde. Keine der Patientinnen erlitt eine ungewollte Frühgeburt. Durch dosisabhängige Verabreichung Nitroglycerinhaltiger Pflaster bis zum gewünschten Geburtstermin war es möglich, heftige Wehentätigkeit zu stoppen oder unregelmäßige Wehentätigkeit zu regulieren.

### Ausführungsbeispiel 1

Das Pflaster wird, wie im Ausführungsbeispiel 1 der EP 0 144 486 B1 ausführlich beschrieben, hergestellt.

Ein pharmazeutisches Produkt gemäß der vorliegenden Erfindung mit dreischichtigem Reservoiraufbau wird folgendermaßen hergestellt:

Eine nitroglycerinhaltige Haftklebemasse wird hergestellt aus

| | |
|---|---|
| 0,175 kg | Polyisobutylen (mittleres Molekulargewicht von 900.000 bis 1.400.000; Handelsprodukt OPPANOL ^{(R)} B 100), |
| 0,157 kg | festes aliphatisches Kohlenwasserstoffharz (Handelsprodukt PICCOTAC CBHT), |
| 0,157 kg | hydriertes Kolophoniumharz (Handesprodukt ABITOL) |
| 0,0105 kg | 5 proz. Lösung von Nitroglycerin in einem mittelkettigen Triglycerid (Handelsprodukt MIGLYOL^{(R)} 812), |
| 1,174 kg | Spezialbenzin 80-110 als Lösungsmittel. |

Das Produkt wird so auf eine einseitig mit Aluminium bedampfte und beidseitig abhäsiv ausgerüstete Schutzschicht aufgetragen, daß nach Abdampfen des Lösungsmittels eine Schicht von ca. 20 g/m² erhalten wird. Auf die so erhaltene Haftklebeschicht wird die erste Reservoirschicht mit einem Flächengewicht von ca. 20 g/m² aufkaschiert.

Die Herstellung dieser Reservoirschicht erfolgt durch Aufbringen einer Dispersion, bestehend aus:

| | |
|---|---|
| 0,05 kg | 10 proz. (G/G) Nitroglycerin-Lactose-Verreibung, |
| 0,153 kg | Polyisobutylen (mittleres Molekulargewicht von 900.000 bis 1.400.000; Handelsprodukt OPPANOL ^{(R)} B 100), |
| 0,137 kg | festes aliphatisches Kohlenwasserstoffharz (Handelsprodukt PICCOTAC CBHT), |
| 0,137 kg | hydriertes Kolophoniumharz (Handesprodukt ABITOL^{(R)}), |
| 0,01 kg | Triglycerid als Lösungsmittel (Handelsprodukt MIGLYOL ^{(R)} 812), |
| 1,148 kg | Spezialbenzin 80 - 110 als Lösungsmittel. |

Das Produkt wird auf ein Trennpapier aufgebracht und das Dispersionsmittel wird sodann abgedunstet.

In analoger Weise wird eine zweite Reservoirschicht mit einem Flächengewicht von ca. 50 g/m² hergestellt aus:

| | |
|---|---|
| 0,6 kg | 10 proz. (G/G) Nitroglycerin-Lactose-Verreibung, |
| 0,2 kg | festes aliphatisches Kohlenwasserstoffharz (Handelsprodukt PICCOTAC CBHT), |
| 0,2 kg | hydriertes Kolophoniumharz (Handesprodukt ABITOL²), |
| 0,025 kg | Triglycerid als Lösungsmittel (Handelsprodukt MIGLYOL ^{(R)} 812), |
| 1,876 kg | Spezialbenzin 80-110 als Lösungsmittel |

und auf die erste Reservoirschicht aufkaschiert.

In analoger Weise wird eine dritte Reservoirschicht hergestellt aus:

| | |
|---|---|
| 2,5 kg | 10 proz. (G/G) Nitroglycerin-Lactose-Verreibung, |
| 0,657 kg | Polyisobutylen (mittleres Molekulargewicht von 900.000 bis 1.400.000; Handelsprodukt OPPANOL ^{(R)} B 100), |
| 0,77 kg | festes aliphatisches Kohlenwasserstoffharz (Handelsprodukt PICCOTAC CBHT), |
| 0,77 kg | hydriertes Kolophoniumharz (Handesprodukt ABITOL^{(R)}), |
| 7,507 kg | Spezialbenzin 80-110 als Lösungsmittel, |

wobei zur Erreichung eines Flächengewichtes von ca. 200 g/m² der Auftrag der Dispersion auf das Trennpapier in 3 aufeinanderfolgenden Schritten erfolgt. Die so erhaltene dritte Reservoirschicht wird auf die zweite Reservoirschicht aufkaschiert.

In analoger Weise wird die haftklebende Zwischenschicht mit einem Flächengewicht von ca. 20 g/m² hergestellt aus:

| | |
|---|---|
| 0,179 kg | Polyisobutylen (mittleres Molekulargewicht von 900.000 bis 1.400.000; Handelsprodukt OPPANOL ^{(R)} B 100), |
| 0,16 kg | festes aliphatisches Kohlenwasserstoffharz (Handelsprodukt PICCOTAC CBHT), |
| 0,16 kg | hydriertes Kolophoniumharz (Handesprodukt ABITOL ^{(R)}), |
| 1,167 kg | Spezialbenzin 80-110 als Lösungsmittel |

und auf die dritte Reservoirschicht aufkaschiert.

Nach Abdecken der haftklebenden Zwischenschicht -mit einer undurchlässigen Deckschicht wird das erhaltene Laminat den therapeutischen Erfordernissen entsprechend in Einzelstücke aufgeteilt.

Das Nitroglycerin-Pflaster besitzt folgenden Aufbau: Es ist ein Matrixsystem, bestehend aus einem mehrschichtig aufgebauten arzneistoffbeladenen Kontaktfilm, aus Polyisobutylen, festem aliphatischem_Kohlenwasserstoffharz und hydriertem Kolophoniumharz, der nach außen durch eine Polyesterfolie abgedeckt wird. Ein Pflaster von 32 cm² enthält 32 mg Glyceroltrinitrat. Die durchschnittliche Wirkstofffreigabe auf der Haut beträgt 10 mg/24 h. Die Freisetzungsfläche des transdermalen Systems wird durch eine silikonisierte und metallisierte Polyesterfolie geschützt, die vor der. Applikation entfernt wird.

### Ausführungsbeispiel 2

Das Pflaster wird, wie im Ausführungsbeispiel, Beispiel 3 der PCT/EP92/01169 = WO 92/22292 A1 ausführlich beschrieben, hergestellt.

Dieses Nitroglycerin-Pflaster besitzt folgenden Aufbau: Es ist ein Matrixsystem, bestehend aus einer Nitroglycerin enthaltenden Klebemasse auf Basis eines vernetzten Acrylat-Vinylacetat-Copolymerisats, das nach außen durch eine Polyesterfolie abgedeckt wird.

Ein derartiges Pflaster von 16 cm² enthält 40 mg Glyceroltrinitrat. Die durchschnittliche Wirkstofffreigabe auf der Haut beträgt 10,8 mg/24 h. Die Freisetzungsfläche dieses transdermalen Systems wird durch eine Polyesterfolie geschützt, die vor der Applikation entfernt wird.

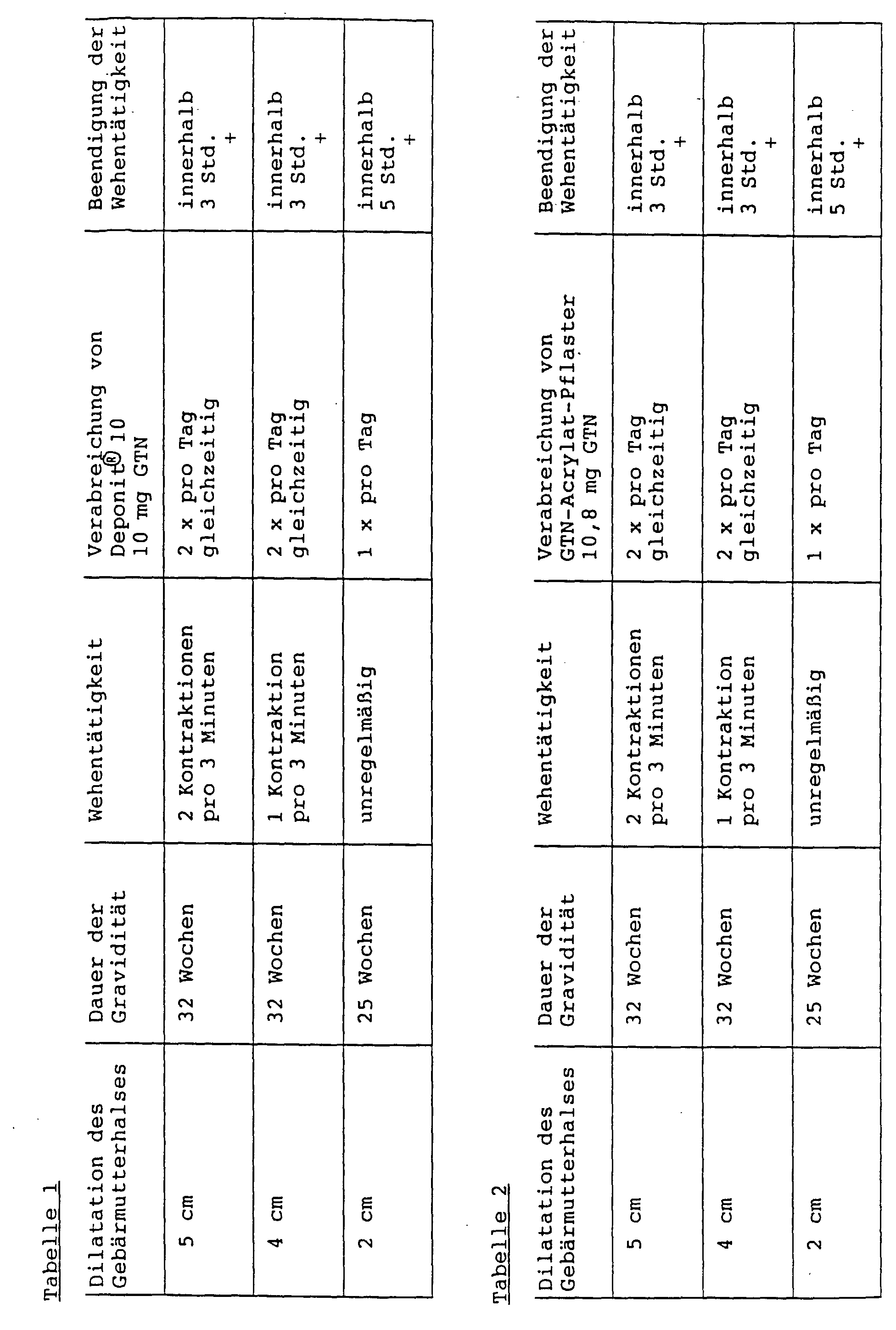

## Patentansprüche

1. Verwendung von 1,2,3-Propantriol-trinitrat zur Herstellung eines Arzneimittels in einer transdermalen Applikationsform zur Verhinderung unerwünschter Wehentätigkeit bei Säugern.

2. Verwendung von 1,2,3-Propantriol-trinitrat nach Anspruch 1 zur Verhinderung von Frühgeburten bei Säugern.

3. Verwendung von 1,2,3-Propantriol-trinitrat zur Herstellung eines Arzneimittels in einer transdermalen Applikationsform zur Regulierung der Wehentätigkeit bei Säugern.

## Claims

1. Use of 1,2,3-propantriol-trinitrate for the production of a medication for transdermal application for the prevention of undesired labour in mammals.

2. Use of 1,2,3-propantriol-trinitrate according to Claim 1 for the prevention of premature births in mammals.

3. Use of 1,2,3-propantriol-trinitrate for the production of a medication for transdermal application for the regulation of labour in mammals.

## Revendications

1. Utilisation de trinitrate de 1,2,3-propanetriol pour la fabrication d'un médicament sous une forme permettant une application transdermique en vue d'empêcher les contractions utérines intempestives chez les mammifères.

2. Utilisation de trinitrate de 1,2,3-propanetriol selon la Revendication 1 en vue d'empêcher les naissances prématurées chez les mammifères.

3. Utilisation de trinitrate de 1,2,3-propanetriol pour la fabrication d'un médicament sous une forme permettant une application transdermique en vue de réguler les contractions utérines chez les mammifères.
